Europäisches Patentamt

European Patent Office

Office européen des brevets

(11) Numéro de publication : **0 365 868 B1**

(19)

# (12) FASCICULE DE BREVET EUROPEEN

(45) Date de publication du fascicule du brevet :
**14.10.92 Bulletin 92/42**

(51) Int. Cl.⁵ : **A61K 37/22,** A23J 7/00,
A61K 9/127, A61K 7/00

(21) Numéro de dépôt : **89118183.6**

(22) Date de dépôt : **30.09.89**

(54) Procédé de séparation des glycolipides d'un mélange lipidique et utilisation des fractions obtenues.

Le dossier contient des informations techniques présentées postérieurement au dépôt de la demande et ne figurant pas dans le présent fascicule.

(30) Priorité : **28.10.88 CH 4033/88**

(43) Date de publication de la demande :
**02.05.90 Bulletin 90/18**

(45) Mention de la délivrance du brevet :
**14.10.92 Bulletin 92/42**

(84) Etats contractants désignés :
**AT BE CH DE ES FR GB GR IT LI LU NL SE**

(56) Documents cités :
**EP-A- 0 237 644**

(56) Documents cités :
**EP-A- 0 242 259**
**DE-A- 2 915 614**
**FR-A- 2 609 393**
**US-A- 4 563 354**
**JOURNAL OF CHROMATOGRAPHY, vol. 153, 1978, NL; K.KROHN et al., pp. 550-552**
**JOURNAL OF CHROMATOGRAPHY, vol. 249, 1982, NL; Z.I.BANDI et al., pp. 93-101**
**SOVIET INVENTIONS ILLUSTRATED, semaine 8738, 30 septembre 1987, no. 87-270011/38, Derwent Publications Ltd, Londres (GB)**

(73) Titulaire : **SOCIETE DES PRODUITS NESTLE S.A.**
**Service des Brevets Case postale 353**
**CH-1800 Vevey (CH)**

(72) Inventeur : **Colarow, Ladislas**
**Eden-Roc 12**
**CH-1073 Savigny (CH)**

## Description

L'invention concerne un procédé de séparation des glycolipides d'un mélange lipidique contenant des lipides complexes (CL).

Les lipides complexes sont des structures chimiques largement répandues dans les tissus biologiques ou ils occupent une place fondamentale dans le métabolisme intermédiaire et dans les cellules de certains organes comme le cerveau et le foie. On les subdivise en deux classes principales: les phospholipides (PL) qui donnent par hydrolyse des phosphates minéraux, par exemple la phosphatidylcholine (PC), la phosphatidyléthanolamine (PE), le phosphatidylinositol (PI); les glycolipides (GL) dont les bases structurelles sphingosine, par exemple les cérébrosides, les hématosides, les gangliosides, glycérol, par exemple les mono- et digalactosyl diglycérides, ou stérols, par exemple les stérylglucosides et leurs esters, contiennent au moins un reste sucre. La quantité de GL dans les CL est généralement 3-10 fois moindre que celle des PL.

Les GL animaux, en concentration élevée dans le cerveau, constituent des composants fonctionnels importants des membranes et sont impliqués dans le transport intramembranaire, la reconnaissance cellulaire, la transmission synaptique, la croissance cellulaire, la fixation des hormones, les enzymes, les bactéries, les toxines bactériennes, les transformations malignes et dans beaucoup d'autres fonctions biologiques. Les GL trouvent ainsi des applications par exemple en médecine, en pharmacologie, en gériatrie et en cosmétique.

On sait extraire partiellement ou totalement les GL des tissus biologiques à l'aide de différents mélanges de solvants organiques polaires, par exemple chloroforme-méthanol, hexane-isopropanol, tétrahydrofuranne-eau. Selon ces procédures, on les obtient seulement en mélange avec les lipides neutres (NL) et les PL et l'on dénomme ces extraits lipides totaux (TL). En variante, on extrait d'abord les tissus biologiques par l'acétone pour en éliminer une grande partie des NL par exemple les triglycérides, les stérols, les acides gras libres, puis on extrait les lipides membranaires résiduels au moyen des solvants organiques polaires mentionnés précédemment. On peut continuer à séparer les TL de plusieurs manières. La méthode la plus répandue est le procédé Folch selon lequel dans un système solvant à deux phases chloroforme-méthanol-eau, on recueille une phase supérieure contenant la plupart des gangliosides, les GL avec plus de 4-5 restes sucres et des traces de PL acides (APL), ainsi qu'une phase inférieure contenant la plupart des PL, les céramides, les stérylglucosides, les céramides mono-, di- et trihexosides et tous les lipides non-polaires, par exemple les triglycérides. Cependant, la séparation des lipides n'est pas nette, par exemple on trouve des hematosides dans les deux phases.

On connaît une méthode de séparation à grande échelle des NL et des CL basée sur l'utilisation d'un mélange hexane-éthanol. D'autres méthodes utilisent la chromatographie liquide, mais présentent l'inconvénient d'une trop faible capacité, par exemple 30 mg de lipides par g de gel de silice. Un procédé fort attrayant décrit dans la demande de brevet DE 2.915.614, consiste à séparer sur gel de silice les TL en les NL et les PL, sans mentionner pour autant les autres CL tels que les gangliosides ou les GL. Dans ce procédé, les NL sont définis comme la partie des TL soluble dans les hydrocarbures par opposition aux PL qui forment des micelles dans les dits solvants. Seuls les lipides formant des micelles sont élués librement du gel de silice alors que les NL restent adsorbés.

Ce procédé présente cependant des inconvénients. En fait beaucoup de GL ne forment pas spontanément des micelles en présence des hydrocarbures. Par suite, il se forme des particules solides insolubles dans les hydrocarbures qui obstruent la colonne de gel de silice qui adsorbe alors les NL. En conséquence, seuls les PL sont élués quantitativement de la colonne.

La récupération complète des GL à partir des mélanges CL est possible mais demande un temps inhabituellement long. Selon la méthode courante, les lipides sont d'abord séchés complètement, pendant 24-48 h, sur le pentoxyde de phosphore, puis acétylés par l'anhydride acétique dans la pyridine. On sépare ensuite le GL acétylés des autres lipides par chromatographie liquide sur colonne de Florisil®, on déacétyle les GL, on les neutralise, on élimine les sels et finalement on sèche par lyophilisation. La méthode est destructive pour tous les PL ainsi que pour certains GL contenant des liaisons sensibles aux bases, par exemple les gangliosides avec acide N-O-diacétylneuraminique. De plus, l'opération nécessite plusieurs jours, voire une semaine.

Dans le but de résoudre les difficultés précédentes, on a proposé une méthode de chromatographie liquide simple, consistant à préparer une résine réutilisable contenant des groupes d'acide phénylboronique (PBA) fixés par liaison covalente sur une matrice de polystyrène réticulé. Cette méthode est décrite dans "Journal of Chromatography", vol.153, (1978), p.550-552. La résine retient sélectivement tous les GL par formation de complexe entre les groupes PBA et les groupes cis-diol de la partie sucre des GL, permettant ainsi une élution complète des PL et des NL. Suit une décomplexation des GL par un solvant organique aqueux. Cependant, la capacité de charge de la résine est relativement faible, typiquement 0,7-7 mg de TL par g ou 0,18 - 1,8 mg par ml de résine (volume apparent). Le volume de solvant d'élution requis pour 1 mg de TL, est de l'ordre de 2,5 -25 ml. Nous avons observé que les cartouches de résine disponibles dans le commerce contenant 100 mg de résine se sont révélées inefficaces pour séparer les PL et les GL.

Nous avons trouvé qu'on pouvait séparer pratiquement quantitativement les GL d'un mélange lipidique contenant des CL par une méthode chromatographique simple non destructive des PL.

Le procédé selon l'invention est caractérisé par le fait que l'on fait passer le mélange en solution dans un solvant approprié et à travers un gel d'acide borique sur support de polyacrylamide réticulé insoluble dans l'eau de manière à adsorber sélectivement les glycolipides, puis que l'on désorbe les glycolipides du gel au moyen d'un solvant l'élution.

Selon l'invention, le mélange lipidique mis en oeuvre peut être d'origine animale ou végétale. Comme lipides animaux, on peut citer ceux provenant de l'extraction par solvant des tissus animaux disponibles aux abattoirs tels que par exemple les intestins, les poumons, le thymus, la moëlle, et de préférence le cerveau bovin. Une autre matière première intéressante est constituée par le placenta humain ou animal. Ces tissus peuvent être dans leur état naturel, congelés ou déshydratés. Une autre matière première intéressante disponible en grandes quantités est constituée par le babeurre doux décaséiné et délactosé. Comme lipides végétaux, on peut citer par exemple les lécithines naturelles de soja.

Pour extraire les lipides de la matière première, on la désagrège et on l'homogénéise, par exemple dans un moulin à grande vitesse dans un solvant ou mélange solvant lipophile mais miscible à l'eau. On peut utiliser par exemple le tétrahydrofuranne, un mélange tétrahydrofuranne: eau de rapport volumique 4:1 - 10:1, un mélange di-isopropyléther:isopropanol de rapport volumique 3:2, les solvants chlorés, par exemple le chloroforme, le dichlorométhane, un mélange chloroforme:méthanol de rapport volumique 2:1. On peut bien entendu utiliser des solvants lipophiles similaires et leurs mélanges. L'opération s'effectue de préférence à chaud, à une température inférieure à 100°C, par exemple à 50-60°C. On filtre ensuite la dispersion, et, après l'élimination des solvants, par exemple par distillation sous vide jusqu'à dessication on recueille les TL bruts. On peut le cas échéant purifier les TL bruts en les redissolvant dans un solvant, puis en décantant et en séparant les particules solides déposées.

Selon une variante d'extraction des lipides, on peut utiliser un solvant hydrocarbure non polaire, par exemple l'hexane, qui est préféré dans le cas d'une matière première constituée de tissus animaux séchés par lyophilisation ou pulvérisation. Dans ce cas, on homogénéise complètement les tissus dans un moulin désintégrateur, par exemple en présence d'hexane à température ambiante, puis on continue l'extraction des TL, par exemple à 60 -62°C sous agitation dans un réacteur fermé muni de moyens d'agitation. On laisse ensuite la suspension au repos à la température ambiante pendant 1-2h. Il se forme un sédiment que l'on sépare du surnageant, puis on retraite le sédiment avec un hydrocarbure non polaire, par exemple l'hexane comme indiqué précédemment. On peut ensuite essorer les surnageants combinés dans une centrifugeuse ou en variante on les laisse au repos pendant 24-48 h à la température ambiante. On sépare le sédiment qui s'est formé, puis on concentre la solution limpide jusqu'à environ 12-15% de son poids initial, par exemple par évaporation sous vide. On peut traiter le concentré de TL en le diluant avec un volume égal d'eau distillée, puis en éliminant l'hexane résiduel, par exemple par évaporation sous azote à 68-70°C et en séchant l'émulsion aqueuse, par exemple par lyophilisation. En variante, on peut utiliser le concentré de TL en solution dans l'hexane directement comme produit intermédiaire de la préparation des CL par chromatographie comme indiqué ci-après. Dans ce cas, on concentre l'éluat contenant les CL jusqu'à 5-10% de son volume initial après élimination du solvant, puis on le dilue avec un volume égal d'eau distillée et on sèche l'émulsion, par exemple par lyophilisation.

Le mélange lipidique peut contenir l'ensemble des lipides, soit les NL et les CL ou, de préférence être constitué des CL seulement. Dans ce cas, on sépare préalablement les CL sur gel de silice dans un solvant hydrocarbure non polaire, non miscible à l'eau, par exemple l'éther de pétrole qui sert de solvant d'élution pour les CL, les NL restant sur la colonne. Lors de la mise en solution des lipides, on préfère employer la sonification de manière à les dissoudre complètement. Les CL recueillis comprennent notamment les PL, les plasmalogènes, les céramidehexosides, les glycéroglycolipides et les gangliosides. Les NL sont désorbés de la colonne en utilisant un mélange solvant lipophile polaire, par exemple un mélange chloroforme:méthanol. Les NL recueillis comprennent notamment les mono-, di- et triglycérides, les acides gras, les tocophérols, les vitamines liposolubles, les oestrogènes et les phytooestrogènes. On voit que les NL ne contiennent pas de GL, ce qui est avantageux lorsqu'on désire recueillir les acides gras et les triglycérides contenant ces acides gras exempts de GL, notamment les acides arachidonique, dihomogammalinolénique, éicosapentaènoïque et docosahexaènoïque présents dans le placenta humain. Après séparation, on élimine les solvants jusqu'à dessication, par exemple par évaporation sous vide.

Selon une variante de préparation du mélange lipidique contenant seulement les CL, plus particulièrement applicable au traitement des lipides du babeurre, on traite le babeurre doux (non acide) décaséiné et délactosé, par exemple par diafiltration (ultrafiltration avec lavage) par un solvant polaire, par exemple l'acétone, de manière à en séparer les NL qui passent, en solution, puis on traite le résidu dont on a éliminé l'acétone par un solvant peu polaire, par exemple le méthanol et on concentre ensuite la solution, par exemple par évaporation du méthanol. Le concentré obtenu peut ensuite être traité sur gel de silice comme indiqué précédemment.

Dans une variante préférée du traitement des lipides du babeurre, on extrait les TL du babeurre délactosé et décaséiné, par exemple par un mélange hexane-méthanol puis, après séparation du résidu, par exemple par micro-filtration ou centrifugation, on élimine ensuite le mélange solvant, par exemple par évaporation. On traite ensuite le concentré de TL par un solvant polaire, par exemple l'acétone, en quantité nettement moindre que dans la variante précédente où le babeurre est directement traité. On recueille la fraction insoluble puis on en élimine l'acétone. Cette variante de pré-séparation présente plusieurs avantages par rapport à la précédente: la quantité d'acétone nécessaire est environ 10-12 fois moindre; la matière insoluble dans l'acétone contient environ 75-80% en poids de CL et environ 20-25% en poids de NL seulement, constitués de triglycérides à haut point de fusion, ce qui nécessite jusqu'à 15 fois moins de gel de silice que dans le cas précédent pour réaliser la séparation ultérieure des CL; enfin, la matière soluble dans l'acétone, représentant environ 40-45% en poids du babeurre mis en oeuvre, comprend, en plus des triglycérides, les acides gras libres, les stérols et la plupart des composés odorants qui peuvent ainsi être éliminés plus aisément. Après cette pré-séparation, on traite le mélange lipidique obtenu par chromatographie sur gel de silice, comme indiqué précédemment.

Pour séparer les PL des GL, on utilise une colonne remplie de gel d'acide borique. L'adsorbant est un polymère réticulé insoluble dans l'eau et les solvants organiques. Il est obtenu par copolymérisation et réticulation de l'acide dihydroxyborylanilino-méthacrylique et du 1,4-butanedioldiméthacrylate. Sans vouloir nous limiter à une théorie, il semble que la séparation pratiquement quantitative des PL et des GL soit due à la faculté de l'adsorbant de former des complexes avec les glycolipides par complexation des cis-diols des GL avec des groupes dihydroxyboryl du gel et décomplexation en présence d'eau. On met le gel d'acide borique en suspension dans un solvant lipophile anhydre moyennement polaire. On peut utiliser par exemple un mélange chloroforme:méthanol de rapport volumique 5:1-2:1, di-isopropylether:isopropanol de rapport volumique 4:2-2:1.

Comme indiqué précédemment, les PL sont élués de la colonne tandis que les GL sont adsorbés. On recueille les PL en utilisant le même solvant l'élution, par exemple le mélange chloroforme:méthanol de rapport volumique 2:1, puis on élimine les solvants par évaporation sous vide jusqu'à dessiccation. Pour désorber les GL de la colonne, on utilise un mélange solvant comprenant un solvant organique polaire miscible à l'eau et 1-25% en volume d'eau désionisée. On peut citer un mélange tétrahydrofuranne:eau de rapport volumique 10:1-4:1, un mélange tétrahydrofuranne:méthanol:eau, un mélange chloroforme:méthanol:eau, un mélange dichlorométhane:méthanol ou éthanol:eau. De préférence, on utilise un mélange tétrahydrofuranne:eau. On peut varier les proportions du mélange au fur et à mesure de l'élution, par exemple commencer avec un mélange riche en solvant organique, typiquement 10:1 en volume et finir avec un mélange 4:1 en volume. Le tétrahydrofuranne présente l'avantage de pouvoir être éliminé en même temps que l'eau facilement, par exemple lors de la lyophilisation subséquente. Si on utilise un mélange contenant un alcool, on ne sortira que les GL solubles dans un mélange alcool:eau et de surcroît il faudra laver la colonne avec un mélange tétrahydrofuranne:eau lors de la régénération.

Dans certains cas, on désire opérer une séparation fine des GL. Pour ce faire, on met les GL en solution dans un mélange solvant chloroforme:méthanol, par exemple dans une ampoule à décanter à double paroi munie de moyens d'agitation à chaud, par exemple 50°C, dans laquelle on ajoute une solution aqueuse de KCl. On agite d'abord rapidement, puis on ralentit l'agitation. Il se forme alors deux phases, une phase aqueuse contenant les gangliosides et une phase organique contenant les autres GL. Après séparation par exemple par soutirage par gravité de la phase inférieure, on en élimine les solvants, par exemple par évaporation sous vide.

Dans un mode de réalisation particulier du procédé selon l'invention, on peut séparer les PL en PL non acides (NPL) et PL acides (APL) d'une part et les GL en GL non acides (NGL) et acides (AGL) d'autre part par chromatographie d'échange d'ions. L'intérêt de ces séparations ultérieures est que les méthodes habituelles d'analyse telles que la chromatographie liquide haute performance (HPLC), la chromatographie haute performance en couche mince (HPTLC) et la spectroscopie de résonance magnétique nucléaire (NMR) ne permettent pas la détection et la détermination quantitative des différentes fractions de CL, car le nombre de classes de lipides présentes dans les extraits biologiques est beaucoup trop élevé pour que ces méthodes soient efficaces. Une élution par HPLC de toutes les classes de lipides présentes ne peut pas être réalisée sans utiliser des phases mobiles différentes ou sans synthétiser des dérivés de certains lipides. La préséparation par résine échangeuse d'ions agarose réticulée, en particulier sur gel de diéthylamino-éthyl-sépharose à débit rapide sous forme acétate permet la séparation quantitative GL et des PL en leurs différentes fractions acides et non acides avec des débits allant jusqu'à 10 volumes de lit/h. Grâce à cette séparation, on peut ensuite identifier et quantifier les différentes fractions par HPTLC combinée à la détection par fluorescence.

Dans un autre mode de réalisation particulier du procédé selon l'invention, on peut traiter des lécithines naturelles de soja par exemple sans séparation préalable des NL, et en éliminer pratiquement les GL et les sucres libres en les faisant passer sur un gel d'acide borique. Une dialyse subséquente de la fraction contenant les GL contre l'eau désionisée permet d'en éliminer les sucres libres. On obtient ainsi des phytoglycolipides. Dans une variante de ce mode de réalisation, on procède à la séparation préalable des NL et des CL sur gel de silice, puis à la séparation des GL et des PL sur gel d'acide borique à partir des CL. Les PL obtenus sont dépourvus de glycolipides et de sucres libres. Ils ne présentent donc plus les risques des PL habituels par exemple dans les émulsions parentérales et dans ce contexte leur composition est comparable à celle de la lecithine d'oeuf.

On peut utiliser les PL, GL et gangliosides de tissus animaux et les PL de babeurre provenant des séparations décrites précédemment dans la préparation de compositions cosmétiques.

On peut utiliser les PL, GL et gangliosides dont la composition est spécifique pour chaque tissu dont ces fractions sont extraites dans la formulation de compositions diététiques destinées notamment à la nutrition infantile.

On peut utiliser les fractions contenant les gangliosides pour la préparation de liposomes utilisables en cosmétique, ou pour la préparation de médicaments pour la thérapeutique du système nerveux.

On peut utiliser les lécithines de soja exemptes de phytoglycolipides dans la préparation d'émulsions parentérales.

Enfin, on peut utiliser les NL de placenta humain pour l'obtention de triglycérides d'acides gras exempts de GL, contenant notamment les acides arachidonique, dihomogammalinolénique, éicosapentaénoïque et docosahexaénoïque. Ces triglycérides peuvent servir, conjointement à d'autres fractions obtenues selon l'invention, notamment les PL, GL et gangliosides à préparer des compositions diététiques destinées notamment à la nutrition infantile.

Les exemples suivants, dans lesquels les parties et pourcentages sont pondéraux sauf indication contraire, illustrent l'invention.

Dans ces exemples, on a vérifié l'efficacité de la séparation des NL et des CL par HPTLC d'échantillons sur plaques de verre de 10 x 10 cm recouvertes de gel de silice. La détection des différents constituants des fractions s'est faite par fluorescence en comparaison avec des composés standards bien définis.

On a établi la séparation quantitative des GL à partir des CL par plusieurs méthodes: HPTLC, colorimétrie et chromatographie en phase gazeuse (GLC). En particulier, la séparation par HPTLC des glycolipides a été établie par détection à l'orcinol qui colore spécifiquement les groupes sucres des glycolipides par comparaison avec des GL purs standards.

Pour analyser la fraction PL, on a recouru à la préséparation des PL en NPL et APL par chromatographie d'échange d'ions, suivie de la détection ultérieure des différents composants par HPTLC de chaque fraction par comparaison avec des PL purs standards.

Parallèlement, on a vérifié le contenu de la fraction GL en procédant à la chromatographie d'échange d'ions préalable, suivie de l'analyse des différents composants non acides (NGL) et acides (AGL) par HPTLC en comparaison avec des échantillons standards.

Exemple 1

1.1. Extraction des TL de cerveau bovin

On homogénéise 5 kg de cerveau bovin en poudre séché par pulvérisation dans 50 l d'un mélange solvant tétrahydrofuranne:eau, de rapport 4:1 en volume à 20°C dans un mélangeur rotatif à 10'000 t/min.

Après 3-5 min, on continue l'extraction des lipides cérébraux dans un réacteur fermé de 100 l avec agitation à 160-250 t/min, à 50-60°C pendant 20 min. On fait passer la suspension obtenue à travers une cuve de filtration à double manteau à 40-50°C munie d'un filtre en papier à un étage. On chasse les particules solides retenus dans la cuve de filtration avec 35 l de mélange tétrahydrofuranne:eau de rapport 4:1 en volume en maintenant le solvant à 40-50°C. On concentre ensuite les filtrats combinés qui contiennent l'extrait lipidique cérébral brut jusqu'à poids constant dans un évaporateur à vide à 65 -75°C, puis on élimine l'eau résiduelle du concentrat lipidique par additions successives de 5 l d'isopropanol suivies d'évaporations azéotropiques.

1.2 Purification des lipides bruts

On redissout l'extrait brut précédent représentant 2,68 kg dans 10 l de tétrahydrofuranne, puis on le centrifuge à 1500-1700 g pendant 10 min dans des bouteilles fermées par des capsules. En variante, on laisse au repos l'extrait brut en solution à la température ambiante pendant 24 h, puis on sépare les solides déposés. On concentre la solution clarifiée jusqu'à la sécher à 65-70°C au moyen d'un évaporateur rotatif sous vide. On obtient 2,32 kg de TL cérébraux et 0,36 kg d'impuretés non lipidiques.

### 1.3 Isolement des CL de cerveau bovin

On remplit une colonne en verre de 100 cm de longueur et 21,3 cm de diamètre intérieur, contenant 15 l d'éther de pétrole à 60-80°C avec 4 kg de particules de gel de silice de 0,21-0,062 mm (70-230 mesh) dispersés dans 20 l d'éther de pétrole. Le gel de silice a été préalablement activé par traitement à 160-165°C pendant au moins 16 h. La colonne en verre comporte un filtre de verre fritté de porosité 100-160 microns de manière à retenir les particules de gel de silice. On dissout les TL cérébraux précédents, soit 2,32 kg par agitation dans 10 l d'éther de pétrole, puis on les sonifie pendant 2-5 min. (250 -500 W/kg de lipides) jusqu'à ce que la solution trouble devienne claire. On maintient la température de l'échantillon sonifié à 45-50°C. On lui fait ensuite traverser la colonne de gel de silice et on élue complètement les CL à l'aide de 15-30 l d'éther de pétrole (éluat 1). On désorbe les NL (éluat 2) du gel de silice en utilisant 15 l d'un mélange de rapport volumique 1:1 de chloroforme:méthanol.

### 1.4 Régénération du gel de silice adsorbant

On chasse 15 l de méthanol, puis 15-20 l d'eau désionisée à travers l'adsorbant. On le sèche ensuite dans une étuve à 105°C pendant 6 h, puis à 160-165°C pendant 18 h. On disperse enfin l'adsorbant réactivé dans 15 l d'éther de pétrole pour éviter tout contact avec l'humidité atmosphérique.

### 1.5 Lyophilisation des CL de cerveau bovin

On concentre l'éluat 1 précédent dans un évaporateur rotatif sous vide de 50-100 l à 50-75°C jusqu'à dessiccation complète. On expose alors les CL à un courant d'azote à température ambiante pendant 12-24 h de manière à éliminer toute trace résiduelle de solvant. On homogénéise ensuite les lipides débarassés de leur solvant dans l'eau distillée (à raison de 5 kg d'eau pour 1 kg de CL), à 40-45°C, puis on étale l'émulsion obtenue en couches de 1-1,5 cm sur des plateaux en acier inoxydable. On congèle alors l'émulsion à -40°C et on la lyophilise. On obtient ainsi 1,45 kg de CL.

### 1.6 Récupération des NL

On concentre l'éluat 2 jusqu'à dessiccation comme au paragraphe 1.5. Après élimination du solvant comme en 1.5, on obtient 0,48 kg de NL.

### 1.7 Séparation des PL et des GL de cerveau bovin

On dissout les CL de cerveau bovin préparés comme indiqué en 1.5 ci-dessus dans un mélange chloroforme:méthanol de rapport 4:1 en volume dans la proportion de 1 g de lipides pour 5 g de mélange solvant. On accélère la dissolution des lipides par sonification à 40-50°C, puis on élimine toute particule en faisant passer la solution sur filtre Büchner.

La colonne d'adsorbtion utilisée en verre, de 30 cm de long et 5 cm de diamètre intérieur, comprend deux supports de lit en verre fritté de 100-160 microns de porosité et un adaptateur de débit permettant de régler la hauteur de lit de 15 à 30 cm. La colonne est reliée à une pompe à basse pression (1-2 bar) qui peut délivrer trois solutions d'élution différente par un robinet à 3 voies à des débits de 0 à 60 ml/min. On met en suspension 150 g de gel d'acide borique dans 1000 ml de tétrahydrofuranne et on verse la suspension dans la colonne par charges successives tout en laissant tout excès de solvant couler par gravité. On ajuste l'adaptateur de débit à la hauteur de lit de 20-22 cm. Avant utilisation, on lave la colonne avec 3 volumes de lit (1200-1300 ml) d'eau désionisée à un débit de 30-50 ml/min, puis avec 1 volume de lit d'acide chlorhydrique 0,5 N, ensuite avec 3 volumes de lit ou plus d'eau désionisée jusqu'à pH neutre, 3 volumes de lit de méthanol et enfin 3 volumes de lit de mélange chloroforme:méthanol de rapport 2:1 en volume.

On prélave la colonne avec 20 ml de mélange chloroforme:méthanol de rapport 4:1 en volume à un débit de 20 ml/min. On charge ensuite la colonne avec l'échantillon de CL de cerveau bovin, soit 30 g de lipides pour 150 g de mélange chloroforme:méthanol de rapport 4:1 en volume, au même débit, puis avec 20 ml de mélange chloroforme:méthanol de rapport volumique 4:1. On élue les PL et les plasmalogènes de la colonne en utilisant 3 volumes de lit (1200-1300 ml) de mélange chloroforme:méthanol de rapport volumique 2:1 au débit de 50 ml/min. Pour éluer tous les GL, comprenant par exemple les céramidehexosides, gangliosides et sulfatides, on utilise 3 volumes de lit de mélange tétrahydrofuranne:eau de rapport volumique 4:1 au même débit que pour les PL.

### 1.8 Régénération en ligne de la colonne de gel d'acide borique

On régénère la colonne de gel d'acide borique après chaque cycle d'élution en utilisant 3 volumes de lit de mélange chloroforme:méthanol de rapport volumique 2:1 au débit de 60 ml/min de manière à déshydrater le gel.

### 1.9 Isolement des PL et des GL de cerveau bovin

On concentre la fraction contenant les PL jusqu'à la sécher sous vide à 65-75°C, puis on la lyophilise comme indiqué précédemment (paragraphe 1.5). On obtient ainsi 950-970 g d'une poudre blanche hygroscopique libre de toute odeur.

On concentre la fraction contenant les GL sous vide à 70-75°C jusqu'à élimination complète du tétrahydrofuranne et on reprend le résidu avec de l'eau désionisée jusqu'à un volume de 2500 ml. Après homogénéisation, on lyophilise l'émulsion contenant les glycolipides comme décrit précédemment. On obtient 480-490 g d'une poudre blanche inodore.

### Exemple 2

### 2.1 Extraction des lipides de babeurre délactosé

On délactose du babeurre frais par diafiltration puis on le sèche par pulvérisation. La poudre de babeurre obtenue a la composition suivante:

|  | % |
|---|---|
| Humidité (mesuré après 2 h d'étuve à 102°C) | 2.5 |
| Matière grasse (méthode Mojonnier) | 52.9 |
| Protéines (N x 6,38) | 29.7 |
| Lactose (déterminé enzymatiquement) | 8.1 |
| Cendres | 6.8 |

On disperse ensuite 80 kg de poudre de babeurre dans 450 l d'acétone à 20-22°C à l'aide d'un homogénéisateur. On extrait la matière grasse dans un réacteur fermé pourvu d'un agitateur à une pale tournant à 120-250 t/min. On maintient la température d'extraction à 20-22°C pendant environ 20 min., puis on filtre la dispersion dans une cuve munie d'un filtre en papier à un étage. On lave alors les particules solides retenues avec 300 l supplémentaires d'acétone à 20-22°C. On sèche ensuite les filtrats combinés par évaporation sous vide et on recueille 26 kg de lipides neutres de babeurre. On fait ensuite passer l'azote à travers le résidu du babeurre dégraissé retenu dans la cuve de filtration maintenue à 35-40°C pendant 2 h pour en éliminer le solvant.

On disperse alors les 53-55 kg de solides du babeurre dégraissé et exempt de solvant dans 560 l de méthanol dans un homogénéisateur rotatif à 10'000 t/min., puis on poursuit l'extraction des lipides dans un réacteur à double paroi à 60-62°C pendant 20-30 min. On fait alors passer la dispersion à travers un filtre en papier disposé dans une cuve à double paroi fermée à 60-62°C sous une pression de 1,5-2,5 bar, puis on chasse les particules solides restées sur le filtre avec 80 l de méthanol chaud. On concentre ensuite le filtrat méthanolique jusqu'à un volume de 40-45 l et on évapore le méthanol résiduel par distillation azéotropique avec 100-150 l d'éther de pétrole qui forme un mélange azéotropique avec le méthanol entre 60-90°C.

### 2.2 Isolement des CL de babeurre

On ajuste le volume du concentré précédent obtenu en 2.1 à 100 l avec de l'éther de pétrole, puis on laisse reposer la solution 48 h à la température ambiante. On élimine les éventuels dépôts solides, par filtration, puis on fait passer la phase d'éther de pétrole clarifiée à travers une colonne de longueur 100 cm et de diamètre intérieur 35 cm contenant 26 kg (correspondant à un volume de lit d'environ 65 l) de gel de silice, comme décrit précédemment à l'exemple 1, paragraphe 3. On élue tous les CL avec 65-70 l d'éther de pétrole (éluat 1), cependant que l'on désorbe les lipides adsorbés avec 80 l d'un mélange de rapport volumique 1:1 de chloroforme:méthanol (éluat 2). On concentre l'éluat 1 sous vide jusqu'à dessiccation, puis on le disperse dans l'eau désionisée et on le lyophilise comme indiqué au paragraphe 5 de l'exemple 1. On obtient ainsi 10,2 kg de CL. L'éluat 2, concentré jusqu'à dessiccation conduit à 5,1 kg de NL résiduels.

### 2.3 Séparation des PL et des GL de babeurre

On fait passer à travers une colonne de gel d'acide borique un échantillon de 600 g de CL de babeurre obtenu au paragraphe 2.2 précédent et on sépare les PL des GL en suivant la procédure indiquée au paragraphe 7 de l'exemple 1. Après concentration jusqu'à dessiccation puis lyophilisation comme indiqué au paragraphe 1.9 de l'exemple 1, on obtient 365 g de PL de babeurre sous forme d'une poudre blanchâtre inodore.

### 2.4 Purification des GL de babeurre

On concentre la fraction contenant les GL sous vide afin d'en éliminer complètement le tétrahydrofuranne comme indiqué au par. 1.9 de l'exemple 1, puis on reprend le résidu avec de l'eau désionisée jusqu'à un volume de 800 ml. On dialyse ensuite la solution aqueuse de GL contre de l'eau désionisée à 4°C pendant 24 h en utilisant une membrane de dialyse ayant une limite de coupure des poids moléculaires de 3500 daltons. On lyophilise alors la solution de GL et on obtient 160 g d'une poudre blanche de GL purifiés contenant moins de 1% de lactose libre. Le dialysat une fois séché représente 75 g.

### Exemple 3

### 3.1 Isolement des CL de moëlle de boeuf

On lyophilise 10 kg de moëlle de boeuf fraîchement homogénéisée et on disperse par homogénéisation le 1,6-1,7 kg de matière sèche obtenue dans 50 l de mélange solvant dichlorométhane:méthanol de rapport volumique 2:1 à 20-22°C. On extrait les TL de moëlle de boeuf dans un réacteur fermé comme à l'exemple 1, par. 1. Après filtration et élimination du mélange solvant comme à l'exemple 1, par. 2, on obtient 0,65 kg de TL que l'on redissout dans 2,5 l d'hexane et que l'on fait passer à travers une colonne en verre de 40 cm de long et 10 cm de diamètre intérieur, remplie avec 750 g de gel de silice, comme indiqué à l'exemple 1.

On élue les CL avec 2 l d'hexane, cependant que l'on désorbe les NL de la colonne en faisant passer 2 l d'un mélange chloroforme:méthanol de rapport volumique 2:1. On lyophilise les CL comme indiqué à l'exemple 1, par. 5 et on obtient 430 g d'une poudre blanche hygroscopique inodore.

### 3.2. Séparation et isolement des GL et des PL de moëlle de boeuf

On dissout les 430 g de CL de moëlle de boeuf précédents dans 2150 g de mélange solvant chloroforme:méthanol de rapport volumique 4:1 comme à l'exemple 1, par. 7. On sépare les CL en leurs fractions PL et GL comme indiqué en 1.7 à partir de 30 g de lipides pour 150 g de solvant et on régénère la colonne de gel d'acide borique après chaque cycle en utilisant 3 volumes de lit de mélange chloroforme:méthanol de rapport volumique 2:1 comme en 1.8. On concentre ensuite sous vide les fractions éluées et désorbées et on les lyophilise comme en 1.9. On obtient ainsi 274 g de PL et 150 g de GL en tant que poudres blanches inodores.

### Exemple 4

#### 4.1 Séparation par solvant des GL de cerveau bovin

On disperse 60 g de GL de cerveau bovin préparé selon l'exemple 1, par. 9 dans un mélange solvant composé de 3200 ml de chloroforme et de 1600 ml de méthanol dans une ampoule à décanter à double paroi de 10 l de capacité munie d'un agitateur à un arbre monté dans sa partie supérieure. L'arbre de l'agitateur est équipé de deux pales distantes l'une de l'autre de sorte que seule la pale inférieure est immergée dans le solvant. On augmente la température de la solution de GL progressivement jusqu'à 50°C, puis on ajoute 1200 ml d'une solution aqueuse de KCl à 88%, ce qui provoque l'immersion de la pale supérieure de l'agitateur. Partant d'une vitesse d'agitation initiale de 300-600 t/min., on ralentit l'agitateur à 5-10 t/min. et on interrompt le chauffage de l'ampoule à décanter après 15 min. Il se forme deux phases qui continuent à être agitées chacune au moyen de l'une des pales. Après 8-12 h, on observe une séparation complète des gangliosides des autres GL lorsque la phase aqueuse supérieure contenant des gangliosides et la phase inférieure contenant les autres GL deviennent claires. On laisse s'écouler la phase inférieure de l'ampoule à décanter par gravité et on recueille séparément la phase supérieure. Les autres GL dans la phase inférieure, sont constitués de monogalactosylcéramides et de sulfatides.

#### 4.2 Isolation des phases de GL

On concentre la phase inférieure qui contient tous les sulfatides et les GL neutres (monogalactosylcéramides) jusqu'à dessiccation sous vide à 65-75°C, puis on la lyophilise comme indiqué à l'exemple 1, par. 9. Le produit final est une poudre blanche immaculée constituée de 49 g de GL purs, (monogalactosylcéramides et sulfatides). On concentre la phase supérieure sous vide à 65-70°C jusqu'à l'élimination complète des solvants organiques dont on réduit ainsi le volume initial d'environ 2400 ml à 300 -600 ml d'une solution claire. On dialyse ensuite la solution aqueuse comme indiqué à l'exemple 2, par. 4, puis on lyophilise la phase supérieure concentrée comme indiqué à l'exemple 1, par. 9. Le produit final est 5,1 g d'une poudre blanche immaculée inodore contenant les gangliosides typiques de cerveau bovin indiqués ci-après, en utilisant les abréviation selon Svennerholm et Holmgren:

NeuAcα2→ 3 Galβ1→ 3 GalNAcβ1→ 4 [NeuAcα2→ 3]Galβ1→ Glc→ Cer (GD1a), NeuAcα2→ 8 NeuAcα2→ 3 Galβ1→ 4Glc→ Cer (GD3), NeuAcα2→ 3 Galβ1→ 3 Gal NAcβ1→ 4[NeuAcα2→ 8 NeuAcα2→ 3] Galβ1→ 4 Glc→ Cer (GT1b) et Galβ1→ 3 Gal NAcβ1→ 4 [NeuAcα2→ 3] Galβ1→ 4 Glc→ Cer (GM1).

### Exemple 5

#### Séparation des GL de babeurre

On sépare les GL de babeurre isolés selon l'exemple 2, par. 4 en utilisant le procédé d'extraction de l'exemple 4. On concentre la phase supérieure contenant les gangliosides sous vide à 65-70°C jusqu'à l'évaporation complète des solvants organiques chloroforme et méthanol, le volume initial d'environ 2400 ml étant ramené à 300-600 ml. On dialyse le concentré aqueux à 4°C pendant 24 h contre l'eau désionisée comme indiqué à l'exemple 2, par. 4. Le produit final est une poudre immaculée de 4,8 g constituée essentiellement des gangliosides typiques de babeurre tels que $GD_3$ (représentant environ 80%), $GM_1$ (explicités à l'exemple 4.2) et NeuAc α 2 → 3 Galβ 1 → 4 Glc → Cer (GM3 selon Svennerholm et Holmgren) Son contenu en lactose libre est inférieur à 1%.

On concentre la phase inférieure jusqu'à dessiccation complète, puis on la lyophilise comme indiqué à l'exemple 4, par. 2. On obtient 46 g d'une poudre blanche immaculée contenant seulement de glycolipides neutres tels que les dihexosyl- et trihexosylcéramides, exempte de lactose libre.

### Exemple 6

#### 6.1 Séparation des CL de placenta humain

On extrait du placenta humain avec un mélange solvant isopropyléther:isopropanol de rapport volumique 3:2, puis on le sonifie dans l'hexane à 55-60°C (400 W/100g de placenta dans 350 ml d'hexane) jusqu'à ce que la solution, au départ trouble, devienne claire. On remplit une colonne de verre de 30 cm de long et 5,5 cm de diamètre intérieur, munie d'un filtre en verre fritté, avec 100 g de particules de gel de silice de 0,21-0,062 mm (70-230 mesh) préalablement activées pendant 15 h à 160-165°C, puis dispersées dans l'hexane.

On fait ensuite passer l'échantillon à 50°C à travers la colonne, puis on complète l'élution des CL par 400-500 ml d'hexane (éluat 1).

On désorbe les NL (éluat 2) de la colonne en utilisant 500 ml de mélange solvant chloroforme:méthanol de rapport volumique 1:1. Après concentration des éluats 1 et 2 jusqu'à dessiccation sous vide on obtient 55,3 g de CL et 44,7 g de NL.

La fraction contenant les CL a l'avantage de ne pas comprendre les oestrogènes qui accompagnent généralement les CL lorsqu'on utilise les méthodes d'extraction et de séparation classiques par mélanges solvants.

Pour régénérer la colonne, on chasse 250 ml de méthanol, puis 1000 ml d'eau désionisée à travers l'adsorbant, puis on l'active thermiquement comme décrit précédemment.

## 6.2 Isolement des PL et des GL de placenta humain

On remplit une colonne de verre de 30 cm de long et 5,5 cm de diamètre intérieur munie d'un filtre en verre fritté avec 100 g de gel d'acide borique. On recouvre le gel en suspension dans un mélange solvant chloroforme:méthanol de rapport volumique 2:1 par une couche de sable de quartz d'épaisseur environ 1 cm. On lave ensuite l'adsorbant successivement avec 3 volumes de lit de méthanol, 3 volumes de lit d'eau désionisée, 1 volume de lit de solution d'HCl 0,5 N, puis 10 volumes de lit d'eau désionisée jusqu'à pH neutre et on met le gel sous forme lipophile en utilisant 3 volumes de lit de méthanol, puis 3 volumes de lit de mélange solvant chloroforme:méthanol de rapport volumique 2:1.

On fait passer à travers la colonne 15 g de CL de placenta humain obtenus comme indiqué en 6.1 dissout dans 60 ml de mélange solvant chloroforme:méthanol de rapport volumique 2:1. Après concentration de l'éluat sous vide jusqu'à dessiccation, on obtient 14 g de PL (éluat 1). On désorbe les GL (éluat 2) de la colonne en utilisant 600 ml de mélange solvant tétrahydrofuranne:eau de rapport volumique 4:1. Après régénération de la colonne par 600 ml de mélange chloroforme:méthanol de rapport volumique 2:1, puis passage d'un second échantillon de 15 g de CL de placenta humain, régénération de la colonne et enfin passage d'un troisième échantillon de 15 g de CL de placenta humain, on combine les fractions de PL ( éluat 1), on en élimine les solvants jusqu'à dessiccation complète et on recueille 40-42 g de PL de placenta humain. Après concentration jusqu'à dessiccation des éluats 2 combinés contenant les GL, on remet le résidu en suspension dans l'eau par sonification à 50°C, puis après lyophilisation, on recueille 3 g de GL de placenta humain.

## Exemple 7

## Isolement des phytoglycolipides de soja

On dissout 25 g de lécithine de soja naturelle du commerce dans 75 ml de mélange solvant chloroforme:méthanol de rapport volumique 4:1, puis on le fait passer sur une colonne de gel d'acide borique comme indiqué à l'exemple 6, par. 2. On obtient ainsi 22,7 g d'un mélange de NL et de PL et 2,3 g d'une fraction contenant les phyto-glycolipides ainsi que les sucres libres. Après dialyse et concentration comme à l'exemple 2, par. 4, on obtient 1 g (4%) de phytoglycolipides de soja exempt de sucres libres.

## Exemple 8

## Séparation des PL et des phyto-glycolipides de soja

On sépare à partir de 100 g de lécithine de soja naturelle du commerce une fraction de 40 g contenant des NL et une fraction de 60 g contenant des CL comme indiqué à l'exemple 1, par. 3.

On redissout la fraction contenant les CL par sonification à 40°C dans 200 ml de mélange solvant diisopropyléther:isopropanol de rapport volumique 3:2, puis on la fait passer par charges successives de 15 g à travers une colonne de gel d'acide borique, dispersé dans un mélange solvant diisopropyléther:isopropanol de 30 cm de long et 5,5 cm de diamètre intérieur. On élue les fractions contenant les PL en utilisant 2 volumes de lit de mélange solvant diisopropyléther:isopropanol de rapport volumique 3:2, puis 1 volume de lit d'isopropanol.

On désorbe les fractions contenant les phyto-glycolipides de la colonne avec 3 volumes de lit de mélange solvant tétrahydrofuranne:eau de rapport volumique 4:1 comme indiqué à l'exemple 6, par. 2. Après chaque cycle, on régénère l'adsorbant en utilisant 1 volume de lit d'isopropanol, puis 2 volumes de lit de mélange solvant diisopropyléther:isopropanol de rapport volumique 3:2.

On recueille séparément les fractions combinées de PL d'une part et de phytoglycolipides d'autre part, on les concentre jusqu'à dessiccation sous vide et on obtient 40 g de PL et 20 g de phytoglycolipides.

Les PL ainsi préparés ont ceci de remarquable qu'ils sont exempts des composés présents naturellement dans les lécithines de soja qui peuvent provoquer des effets secondaires indésirables.

Ils sont comparables à ceux de la lécithine de jaune d'oeuf qui sont pauvres en glycolipides et sucres libres ce qui les rend attractifs pour préparer des émulsions parentérales bien tolérées par l'organisme humain.

Exemple 9

9.1 Séparation des PL et des GL d'intestin de cochon d'Inde

On lave, puis homogénéise 7,86 g d'intestins de cochon d'Inde successivement dans 2 x 80 ml de mélange solvant chloroforme:méthanol de rapport volumique 1:2, on centrifuge l'homogénéisat à 2500 g et on concentre le surnageant clair sous vide jusqu'à dessiccation.

On dissout les 290 mg de lipides bruts obtenus par sonification dans 10 ml de tétrahydrofuranne, on centrifuge la solution à 2500 g et on la concentre jusqu'à dessiccation pour obtenir 250 mg de TL. On élimine les NL comme les stérols, acides gras libres et triglycérides en dissolvant les TL par sonification dans 2 x 2,5 ml d'hexane et en faisant passer la solution à travers une colonne en verre de 10 cm de long et 1 cm de diamètre intérieur remplie de particules de gel de silice activé de 0,21-0,062 mm (70-230 mesh). On élue ainsi quantitativement 190 mg de CL avec 25 ml d'hexane. On désorbe 57 mg de NL de la colonne par 3 volumes de lit de mélange solvant chloroforme:méthanol de rapport volumique 2:1. On sépare ensuite les CL en 143 mg de PL et 47 mg de GL comme indiqué précédemment à l'exemple 1, par. 7. Après dialyse contre l'eau désionisée, puis lyophilisation, on obtient 16,6 mg d'une fraction de GL purs sans sucres libres.

9.2 Séparation des PL d'intestin de cochon d'Inde en NPL et APL par échange d'ions

On suspend 8,5 ml de gel de diéthylaminoéthyl-sépharose à débit rapide dans l'éthanol et on en remplit une colonne en verre de 10 cm de long et 1,15 cm de diamètre intérieur. On lave ensuite le gel avec 30 ml d'eau désionisée, puis on le met sous forme acétate avec 20 ml d'une solution aqueuse d'acétate de sodium 2M, et on le lave successivement avec 30 ml d'eau désionisée, 10 ml de méthanol et enfin 10 ml de mélange solvant chloroforme:méthanol de rapport volumique 2:1. On recouvre le gel d'une couche de 7-10 mm de sable de quartz. On fait passer l'échantillon de 143 mg de PL d'intestin de cochon d'Inde à travers la colonne, puis on élue les PL non acides avec des portions successives de 2 x 2 ml, puis 10 ml de mélange solvant chloroforme:méthanol de rapport volumique 2:1 et enfin 5 ml de méthanol. On maintient une légère pression de manière à accélérer l'élution. Après élimination des solvants sous vide jusqu'à dessiccation, on obtient 107 mg de NPL.

Pour désorber le PL acides retenus sur la colonne, on fait passer à travers celle-ci 15 ml de mélange solvant chloroforme-méthanol-solution aqueuse d'acétate de sodium 0,8M (30:60:8). Après concentration de la fraction acide jusqu'à dessication, redissolution de celle-ci par sonification dans 2 x 1 ml de mélange solvant chloroforme:méthanol de rapport volumique 2:1, on élimine les sels sur une colonne en verre de 10 cm de long et 1,15 cm de diamètre intérieur remplie avec 4 ml de gel Sephadex G-25 dans un mélange solvant chloroforme:méthanol:eau de rapport volumique 60:30:4,5, en maintemant la colonne sous légère pression. Après élimination des solvants sous vide jusqu'à dessiccation, on obtient 35 mg de APL. Après chaque cycle, on régénère la colonne échangeuse d'ions en utilisant 1 volume de lit de méthanol, puis 2 volumes de lit de mélange solvant chloroforme:méthanol de rapport volumique 2:1.

La fraction de NPL comprend typiquement la phosphatidylcholine, la phosphatidyléthanolamine, la sphingomyéline.

La fraction de APL comprend typiquement le phosphatidyl-inositol, la phosphatidylsérine, l'acide phosphatidique, la cardiolipine.

9.3 Séparation des GL d'intestin de cochon d'Inde en NGL et AGL

Pour séparer les fractions NGL et AGL, on utilise la méthode décrite au par. 9.2 précédent. On obtient à partir de 16,6 mg précédents (par. 9.1) 11,9 mg de NGL et 4,5 mg de AGL.

Les NGL obtenus sont typiquement les céramidehexosides, et les glycoglycérolipides.

Les AGL obtenus sont typiquement les sulfatides, les hématosides et les gangliosides.

Exemple 10

10.1. Variante d'extraction des TL de cerveau bovin

On homogénéise 8 kg de cerveau bovin en poudre séché par pulvérisation à température ambiante dans 45 kg d'hexane dans un moulin colloïdal, puis on continue l'extraction à 60°C dans un réacteur fermé muni d'agitateurs à pales tournant à 250 t/min.

Après 30 min. d'extraction, on laisse la suspension reposer pendant 1-2 h dans un récipient séparé. On recueille le surnageant par décantation et on ré-extrait les solides ayant sédimenté avec 30 kg d'hexane supplémentaires.

On laisse le deuxième extrait au repos pendant 1-2 h, on recueille le surnageant par décantation et on le combine avec le surnageant précédent. Après avoir reposé 24-48 h à la température ambiante, les surnageants combinés conduisent à une phase liquide translucide dont on sépare un résidu solide par décantation soigneuse.

On préconcentre ensuite la phase liquide à 66-70°C jusqu'à 12-15% de son volume initial par évaporation sous vide, puis on dilue le préconcentré avec un volume égal d'eau distillée. On élimine le solvant par distillation à 68-70°C, on homogénéise l'émulsion obtenue, puis on la congèle à -40°C et on la lyophilise. On obtient ainsi 3,32kg de TL de cerveau bovin sous forme d'une poudre beige hygroscopique inodore de composition:

|  | % |
|---|---|
| Cholestérol | 25-27 |
| CL dont | 73-75 |
| PL | 50-53 |
| GL dont | 20-23 |
| GL neutres et sulfatides | 15-18 |
| Gangliosides | 1,5-1,8 |

10.2. Variante de séparation des CL et des NL de cerveau bovin

On procède comme en 10.1 ci-dessus au traitement de 8 kg de poudre de cerveau bovin jusqu'à la préconcentration de la phase liquide. Le concentré dans l'hexane contient 3,3 kg de TL. On réalise une dispersion fine par sonification, puis on fait passer la dispersion à travers une colonne semblable à celle décrite à l'exemple 1, paragraphe 3 chargée avec 3,3-3,5 kg de gel de silice. On élue ensuite les CL de la colonne avec 20-25 l d'hexane, puis on préconcentre l'éluat jusqu'à 12-15% de son poids initial par évaporation de l'hexane sous vide. Après dilution avec le même poids d'eau, on traite l'émulsion comme en 10.1 ci-dessus, ce qui conduit à 2,45 kg de CL contenant:

|  | % |
|---|---|
| PL | 70-73 |
| GL dont | 25-28 |
| GL neutres et sulfatides | 20-25 |
| Gangliosides | 2-3 |

Pour désorber les NL de la colonne, on utilise 2 volumes de lit de mélange chloroforme: méthanol en proportions volumiques 2:1, puis on concentre l'éluat jusqu'à 5% de son propre poids initial par évaporation sous vide. Après dilution avec 5 l d'hexane, on concentre la solution de NL jusqu'à siccité et on obtient 0,88 kg d'une poudre jaune contenant plus de 95% de cholestérol.

Exemple 11

11.1 Variante de séparation des CL de babeurre

On décaséine et délactose du babeurre doux par diafiltration, puis on le sèche par pulvérisation. La poudre de babeurre obtenue a la composition suivante:

|  | % |
|---|---|
| Humidité (après 2h d'étuve à 102°C) | 3,22 |
| Solides totaux, dont | 96,78 |
| Lipides totaux (méthode Mojonnier) | 61,24 |
| Protéines (N x 6,38) | 32,43 |
| Lactose (déterminé enzymatiquement) | 1,24 |
| Cendres | 1,87 |

On homogénéise 100 g de cette poudre en 2 fois, chaque fois avec 500 ml d'un mélange hexane:méthanol de rapport volumique 94:6 pendant 1 min. à 50°C dans un moulin colloïdal. A chaque fois, on essore l'homogénéisat pendant 2 min. à 1500 g.

On concentre ensuite le surnageant jusqu'à siccité sous vide à 65°C et on obtient ainsi 65,2 g de TL qui restent fluides à cause de leur haut pourcentage d'huile de beurre (70-75%).

On refroidit les TL à 40°C, on les homogénéise avec 1,5 -2 fois leur volume d'acétone (90-130 ml) dans un moulin colloïdal, on les laisse atteindre la température ambiante (22-24°C), puis on les centrifuge pendant 1 min. à 2000 g. On élimine le surnageant limpide contenant la plupart des NL et des composés odorants. Le résidu solide recueilli contient les CL. On les redissout dans l'hexane, puis on le concentre par évaporation sous vide. On obtient ainsi 21,7 g de matière insoluble dans l'acétone (AIM).

On suspend cette matière dans trois fois son volume d'hexane, puis on en sépare les CL (80,6%) des NL (19,4%)

Par chromatographie liquide d'adsorption en utilisant un volume équivalent de gel de silice (10-11 g).

On homogénéise les CL de babeurre précédents (17,5 g) dans 10 fois leur volume d'eau distillée et on lyophilise l'émulsion.

11.2. Seconde variante de séparation des CL de babeurre

On traite 7,84 kg de poudre de babeurre de composition indiquée ci-dessus en 11.1 par 90 l de mélange solvant hexane:méthanol en proportions volumiques 94:6 au reflux sous agitation. Après 15 min., on refroidit la suspension obtenue à 40°C, puis on la fait passer à travers un microfiltre, après quoi on lave le filtre avec 10 l de mélange hexane-méthanol de rapport volumique 94:6.

On concentre ensuite le filtrat jusqu'à siccité à 65°C par évaporation sous vide et on obtient 4,57 kg de TL que l'on refroidit à 40°C, homogénéise avec 2 fois leur volume d'acétone (10 l) et laisse refroidir à la température ambiante (22-24°C).

Après centrifugation pendant 1-3 min. à 1500 g, on met de côté le surnageant limpide contenant la plupart des LN et des composés odorants, on reprend la matière insoluble dans l'acétone (AIM) par 10 l d'hexane, puis on la concentre jusqu'à siccité par évaporation sous vide. On obtient ainsi 1,52 kg d'AIM.

On reprend l'AIM dans 3-4 fois son volume d'hexane (6 -7 l), puis on la sépare en ses CL (1,16 kg) et ses NL (0,36 kg) par chromatographie d'adsorption sur gel de silice en utilisant environ 1 kg d'adsorbant. On recueille l'éluat contenant les CL que l'on sèche jusqu'à siccité par évaporation sous courant d'azote. Après homogénéisation pendant 5 min. à 40°C avec 9 fois son volume d'eau distillée, on sèche l'émulsion par lyophilisation.

## Revendications

1. Procédé de séparation des glycolipides d'un mélange lipidique contenant des lipides complexes, caractérisé par le fait que l'on fait passer le mélange en solution dans un solvant approprié à travers un gel d'acide borique de manière à adsorber sélectivement les glycolipides, puis que l'on désorbe les glycolipides du gel au moyen d'un solvant d'élution.

2. Procédé selon la revendication 1, caractérisé par le fait que le mélange lipidique mis en oeuvre est un mélange de lipides complexes obtenu après séparation des lipides neutres sur gel de silice et contenant des glycolipides et des phospholipides exempts d'oestrogènes.

3. Procédé selon la revendication 1, caractérisé par le fait que l'on fait passer les glycolipides sur une résine échangeuse d'ions de manière à séparer les glycolipides acides des glycolipides non acides.

4. Procédé selon la revendication 1, caractérisé par le fait que l'on fait passer les phospholipides sur une résine échangeuse d'ions de manière à séparer les phospholipides acides des phospholipides non acides.

5. Procédé selon la revendication 1, caractérisé par le fait que le mélange lipidique mis en oeuvre provient de l'extraction par solvant de tissus animaux, notamment de cerveau, de thymus ou de moëlle de bovins.

6. Procédé selon la revendication 1, caractérisé par le fait que le mélange lipidique mis en oeuvre provient de l'extraction par solvant du babeurre gras délactosé.

7. Procédé selon la revendication 6, caractérisé par le fait que l'on traite le babeurre par un solvant polaire de manière à en séparer les lipides neutres qui passent en solution, puis après avoir récupéré le résidu et en avoir éliminé le solvant polaire, on le traite par un solvant peu polaire puis, après avoir éliminer ce solvant peu polaire, on sépare le mélange lipidique contenant les lipides complexes par chromatographie d'adsorption sur gel de silice.

8. Procédé selon la revendication 6, caractérisé par le fait que l'on traite d'abord le babeurre par un solvant peu polaire, puis après avoir séparé le résidu, on recueille la solution dont on élimine le solvant de manière à obtenir un concentré, on traite ensuite le concentré par un solvant polaire, puis on recueille la fraction insoluble, on en élimine le solvant polaire et on sépare le mélange lipidique contenant les lipides complexes par chromatographie d'adsorption sur gel de silice.

9. Procédé selon la revendication 1, caractérisé par le fait que le mélange lipidique mis en oeuvre provient de l'extraction par solvant du placenta humain ou animal.

10. Procédé selon la revendication 1, caractérisé par le fait que le mélange lipidique mis en oeuvre provient de l'extraction par solvant d'intestins animaux.

11. Procédé selon la revendication 1, caractérisé par le fait que le mélange lipidique mis en oeuvre est une lécithine naturelle de soja.

12. Procédé selon la revendication 1, caractérisé par le fait que l'on sépare les gangliosides des autres glycolipides par extraction par solvant des glycolipides en présence d'eau, les gangliosides se retrouvant dans la phase aqueuse.

## Patentansprüche

1. Verfahren zum Abtrennen von Glykolipiden aus einem Lipidgemisch mit einem Gehalt an komplex gebundenen Lipiden, dadurch gekennzeichnet, daß man das in Lösung in einem geeigneten Lösungsmittel befindliche Gemisch durch ein Borsäuregel auf einem wasserunlöslichen Träger aus vernetztem Polyacrylamid führt, um die Glykolipide selektiv zu adsorbieren, und daß man anschließend die Glykolipide von dem Gel mit Hilfe eines Elutionslösungsmittels desorbiert.

2. Verfahren nach Anspruch 1, dadurch gekennzeichnet, daß das eingesetzte Lipidgemisch ein Gemisch komplex gebundener Lipide ist, welches nach Abtrennen der neutralen Lipide auf Silikagel erhalten worden ist, und welches von Östrogenen freie Glykolipide und Phospholipide enthält.

14

3. Verfahren nach Anspruch 1, dadurch gekennzeichnet, daß man die Glykolipide über ein Ionenaustauscherharz führt, um die sauren Glykolipide von den nicht-sauren Glykolipiden abzutrennen.

4. Verfahren nach Anspruch 1, dadurch gekennzeichnet, daß man die Phospholipide über ein Ionenaustauscherharz führt, um die sauren Phospholipide von den nicht-sauren Phospholipiden abzutrennen.

5. Verfahren nach Anspruch 1, dadurch gekennzeichnet, daß das eingesetzte Lipidgemisch aus der Lösungsmittelextraktion von tierischen Geweben, insbesondere von Rinderhin, -thymus oder -knochenmark bzw. -rückenmark, stammt.

6. Verfahren nach Anspruch 1, dadurch gekennzeichnet, daß das eingesetzte Lipidgemisch aus der Lösungsmittelextraktion von fetter, von Lactose befreiter Buttermilch stammt.

7. Verfahren nach Anspruch 6, dadurch gekennzeichnet, daß man die Buttermilch mit einem polaren Lösungsmittel behandelt, um daraus die neutralen Lipide abzutrennen, welche in Lösung gehen, und daß man anschließend, nachdem man den Rückstand zurückgewonnen und das polare Lösungsmittel daraus entfernt hat, denselben mit einem wenig polaren Lösungsmittel behandelt, und daß man, nachdem man dieses wenig polare Lösungsmittel eliminiert hat, das die komplex gebundenen Lipide enthaltende Lipidgemisch durch Adsorptionschromatographie auf Silikagel auftrennt.

8. Verfahren nach Anspruch 6, dadurch gekennzeichnet, daß man die Buttermilch zuerst mit einem wenig polaren Lösungsmittel behandelt, und daß man, nachdem man den Rückstand abgetrennt hat, die Lösung sammelt, aus der man das Lösungsmittel entfernt, um ein Konzentrat zu erhalten, daß man anschließend das Konzentrat mit einem polaren Lösungsmittel behandelt, daß man dann die unlösliche Fraktion sammelt, daß man daraus das polare Lösungsmittel entfernt, und daß man das die komplex gebundenen Lipide enthaltende Lipidgemisch durch Adsorptionschromatographie auf Silikagel auftrennt.

9. Verfahren nach Anspruch 1, dadurch gekennzeichnet, daß das eingesetzte Lipidgemisch aus der Lösungsmittelextraktion von Human-Plazenta oder tierischer Plazenta stammt.

10. Verfahren nach Anspruch 1, dadurch gekennzeichnet, daß das eingezetzte Lipidgemisch aus der Lösungsmittelextraktion von Tiergedärmen stammt.

11. Verfahren nach Anspruch 1, dadurch gekennzeichnet, daß das eingesetzte Lipidgemisch ein natürliches Sojalecithin ist.

12. Verfahen nach Anspruch 1, dadurch gekennzeichnet, daß man die Ganglioside von den anderen Glykolopiden durch Lösungsmittelextraktion der Glykolipide in Gegenwart von Wasser abtrennt, wobei sich die Ganglioside in der wässerigen Phase wiederfinden.

## Claims

1. A process for the separation of glycolipids from a lipid mixture containing complex lipids, characterized in that the mixture dissolved in a suitable solvent is passed over a boric acid gel on a support of crosslinked water-insoluble polyacrylamide so that the glycolipids are selectively adsorbed, after which the glycolipids are desorbed from the gel with an elution solvent.

2. A process as claimed in claim 1, characterized in that the lipid mixture used is a mixture of complex lipids obtained after separation of the neutral lipids on silica gel and containing glycolipids and phospholipids free from oestrogens.

3. A process as claimed in claim 1, characterized in that the glycolipids are passed over an ion exchange resin to separate the acidic glycolipids from the non-acidic glycolipids.

4. A process as claimed in claim 1, characterized in that the phospholipids are passed over an ion exchange resin to separate the acidic phospholipids from the non-acidic phospholipids.

5. A process as claimed in claim 1, characterized in that the lipid mixture used emanates from the solvent extraction of animal tissues, particularly bovine brain, thymus or bone marrow.

6. A process as claimed in claim 1, characterized in that the lipid mixture used emanates from the solvent extraction of lactose-free whole buttermilk.

7. A process as claimed in claim 6, characterized in that the buttermilk is treated with a polar solvent to separate the neutral lipids which pass into solution and, after the residue has been recovered and the polar solvent eliminated, the residue is treated with a slightly polar solvent and, after this slightly polar solvent has been eliminated, the lipid mixture containing the complex lipids is separated by adsorption chromatography on silica gel.

8. A process as claimed in claim 6, characterized in that the buttermilk is first treated with a slightly polar solvent and, after the residue has been separated, the solution is collected and the solvent eliminated to obtain a concentrate, after which the concentrate is treated with a polar solvent and the insoluble fraction is collected, the polar solvent is eliminated therefrom and the lipid mixture containing the complex lipids is separated by adsorption chromatography on silica gel.

9. A process as claimed in claim 1, characterized in that the lipid mixture used emanates from the solvent extraction of human or animal placenta.

10. A process as claimed in claim 1, characterized in that the lipid mixture used emanates from the solvent extraction of animal intestines.

11. A process as claimed in claim 1, characterized in that the lipid mixture used is a natural soya lecithin.

12. A process as claimed in claim 1, characterized in that the gangliosides are separated from the other glycolipids by solvent extraction of the glycolipids in the presence of water, the gangliosides reappearing in the aqueous phase.